(19) Europäisches Patentamt
European Patent Office
Office européen des brevets

(11) Publication number: **0 002 058**
**B1**

(12) **EUROPEAN PATENT SPECIFICATION**

(45) Date of publication of patent specification: **23.06.82**

(21) Application number: **78101384.2**

(22) Date of filing: **16.11.78**

(51) Int. Cl.$^3$: **C 07 D 487/04,**
C 12 P 17/10, A 61 K 31/40
//(C07D487/04, 209/00,
205/00)

(54) **3-(2-Aminoethylthio-6-ethyl-7-oxo-1-azabicylo-(3.2.0)-hept-2-en-2-carboxylic acid, process for preparing it and compositions comprising it.**

(30) Priority: **17.11.77 JP 137276/77**

(43) Date of publication of application:
**30.05.79 Bulletin 79/11**

(45) Publication of the grant of the patent
**23.06.82 Bulletin 82/25**

(84) Designated Contracting States:
**DE FR GB**

(56) References cited:
**DE - A - 2 815 157**

(73) Proprietor: **SANRAKU-OCEAN CO., LTD.**
**15-1, Kyobashi 1-chome Chuo-ku**
**Tokyo (JP)**

(72) Inventor: **Okamura, Kazuhiko**
**2412 Kamiwada**
**Yamato-shi Kanagawa-ken (JP)**
Inventor: **Hirata, Shoji**
**51-8, Aza Tateminami Watari-cho**
**Watari-gun Miyagi-ken (JP)**
Inventor: **Okumura, Yasushi**
**534-1, Ueki**
**Kamakura-shi Kanagwa-ken (JP)**
Inventor: **Fukagawa, Yasuo**
**1194-146 Imaizumi**
**Kamakura-shi Kanagawa-ken (JP)**
Inventor: **Shimauchi, Yasutaka**
**1-101-13, Yurigaoka**
**Ninomiya-machi Kanagawa-ken (JP)**
Inventor: **Ishikura, Tomoyuki**
**640, Kowada**
**Chigasaki-shi Kanagawa-ken (JP)**
Inventor: **Kouno, Kageaki**
**7-16-3, Ikegami Ohta-ku**
**Tokyo (JP)**
Inventor: **Lein, Joseph**
**4, Thornwood LN**
**Fayetteville New York (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 002 058 B1**

Courier Press, Leamington Spa, England.

(74) Representative: **Kraus, Walter, Dr. et al,**
**Patentanwälte Dres. Kraus & Weisert**
**Irmgardstrasse 15**
**D-8000 München 71 (DE)**

# 3-(2-Aminoethylthio-6-ethyl-7-oxo-1-azabicyclo-(3.2.0)-hept-2-en-2-carboxylic acid, process for preparing it and compositions comprising it

This invention relates to a new antibiotic derivative, its preparation and to its use as an antimicrobial agent.

Some antibiotics having β-lactamase inhibitory activity or β-lactamase inhibitory agents have so far been known. For example, the following have been reported: MC696—SY2 and B isolated from the culture broth of an MC696—SY2 producing strain belonging to the genus *Streptomyces* (U.S. Patent 3,928,569: Derwent CPI 19171X), MM 4550 or MM 13902 isolated from a cultivation product of an MM 4550 or MM 13902 producing strain which belongs to the genus *Streptomyces* (German Appln. DOS 2,513,855: Derwent CPI 67721W; German Appln. DOS 2,513,854: Derwent CPI 67720W), clavulanic acid isolated from a cultivation product of *Streptomyces clavuligerus* (German Appln. DOS 2,517,316: Derwent CPI 72840W). The antibiotic thienamycin having a penicillin-like chemical skeleton and the derivatives thereof have also been reported (U.S. Patent 3,950,357: Derwent CPI 31696X; German Appln. DOS 2,652,677: Derwent CPI 40282Y; Belgian Patent 848,346: Derwent CPI 34505Y; Belgian Patent 848,349: Derwent CPI 34507Y; German Appln. DOS 2,652,680: Derwent CPI 40283Y; German Appln. DOS 2,652,675: Derwent CPI 40280Y; German Appln. DOS 2,652,674: Derwent CPI 40279Y; German Appln. DOS 2,652,676: Derwent CPI 40281Y).

The present inventors discovered an antibiotic β-lactam antibiotic "PS—5", of the formula

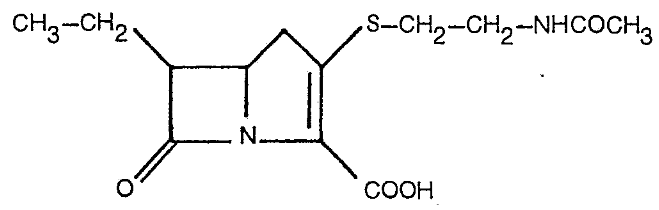

(IV)

which is not described in the aforesaid prior literature references, and already disclosed it in Japanese Patent Applications Nos. 35375/77 and 94651/77.

SUMMARY OF THE INVENTION

This invention relates to a new antibiotic derivative

More particularly, it is concerned with a novel antibiotic derivative defined as "PS—5 N-desacetyl derivative", which has strong antibiotic activity and β-lactamese inhibitory activity against Gram-positive and Gram-negative bacteria including β-lactam antibiotics-resistant strains. It also relates to a method for producing the novel antibiotic derivative and to an antibiotic composition containing it.

The first object of the present invention is to provide the new antibiotic substances PS—5 N-desacetyl derivative having both strong antibiotic activity and β-lactamase inhibitory activity.

Another object of this invention is to provide a method for producing the antibiotic PS—5 N-desacetyl derivative by a chemical or biological method.

A further object of this invention is to provide preventive and therapeutic methods and compositions of the antibiotic PS—5 N-desacetyl derivative for use in infectious diseases caused by Gram-positive and Gram-negative bacteria, including synergic combinations with penicillins, cephalosporins and other β-lactamase sensitive antibiotics.

Other objectives of the invention will become apparent from the following description.

Detailed Description Of The Invention

According to this invention, in the course of studies on derivatives of the antibiotic PS—5 having formula (IV) mentioned above, the present inventors have discovered that the N-acetyl group can be easily cleaved off by a chemical or biological method and that the resultant N-desacetyl derivative of the new antibiotic PS—5 shows a high antimicrobial activity against Gram-positive and Gram-negative bacteria. It has also been found that the antimicrobial activity of the derivative on staphylococci is far higher than that of the antibiotic PS—5 itself.

Thus, according to this invention there is first provided a compound of the formula (I), particularly a compound having 5,6-trans stereo configuration of the formula (I)

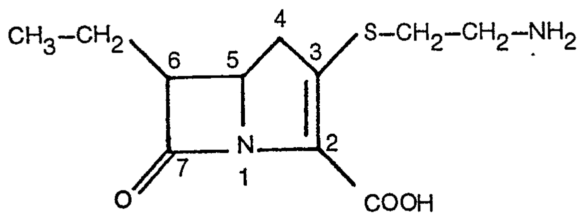

(I)

It is presumed that the compound of formula (I) may be present in the form of a zwitterion of the following formula (I-a)

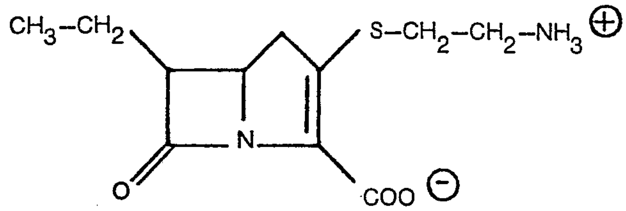

(I-a)

In this specification and appended claims, the above formula (I) includes both the N-desacetyl derivatives of the antibiotic PS—5 and the compound of the zwitterion-type shown by the formula (I-a). In the present application, the compound of formula (I) is sometimes referred to as (antibiotic) PS—5 N-desacetyl derivative.

The compound of formula (I) according to this invention shows a high antimicrobial activity on a wide variety of Gram-positive and Gram-negative bacteria including β-lactam antibiotics-resistant strains.

Particularly on staphylococci, the antimicrobial activity of antibiotic PS—5 N-desacetyl derivative is much superior to that of the antibiotic PS—5 itself. The inhibitory activity of the derivative on *Staphylococcus aureus* FDA 209p, for example, is 10 times higher than that of the antibiotic PS—5. This is an extraordinarily strong antimicrobial activity.

According to the present invention, the compound of formula (I), which possesses such an excellent antimicrobial activity, can be produced by cleaving off the N-acetyl group from the antibiotic PS—5 of formula (IV), salts thereof or carboxyl-protected derivatives thereof, by a chemical or biological method.

According to one aspect of this invention, the compound of formula (I) or carboxyl-protected derivatives thereof are produced by a method which comprises reacting a compound of the formula

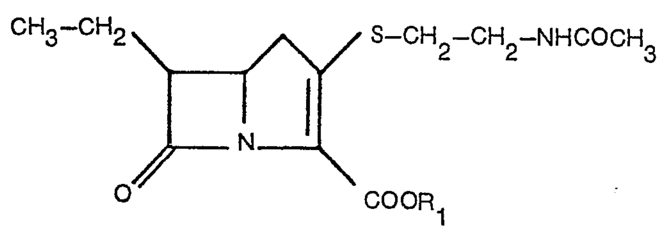

(II)

wherein $R_1$ represents a protecting group for the carboxyl group, with an iminohalogenating agent, and reacting the resulting iminohalide compound with an alcohol of the formula

$$R_2—OH \qquad (III)$$

wherein $R_2$ represents a hydrocarbon residue, followed by treatment with water, and if desired splitting off the carboxyl-protecting group, which may be present, from the reaction product.

The reaction mechanism of this process is not clearly understood but it is presumed that the reactions proceed in accordance with the following reaction formula

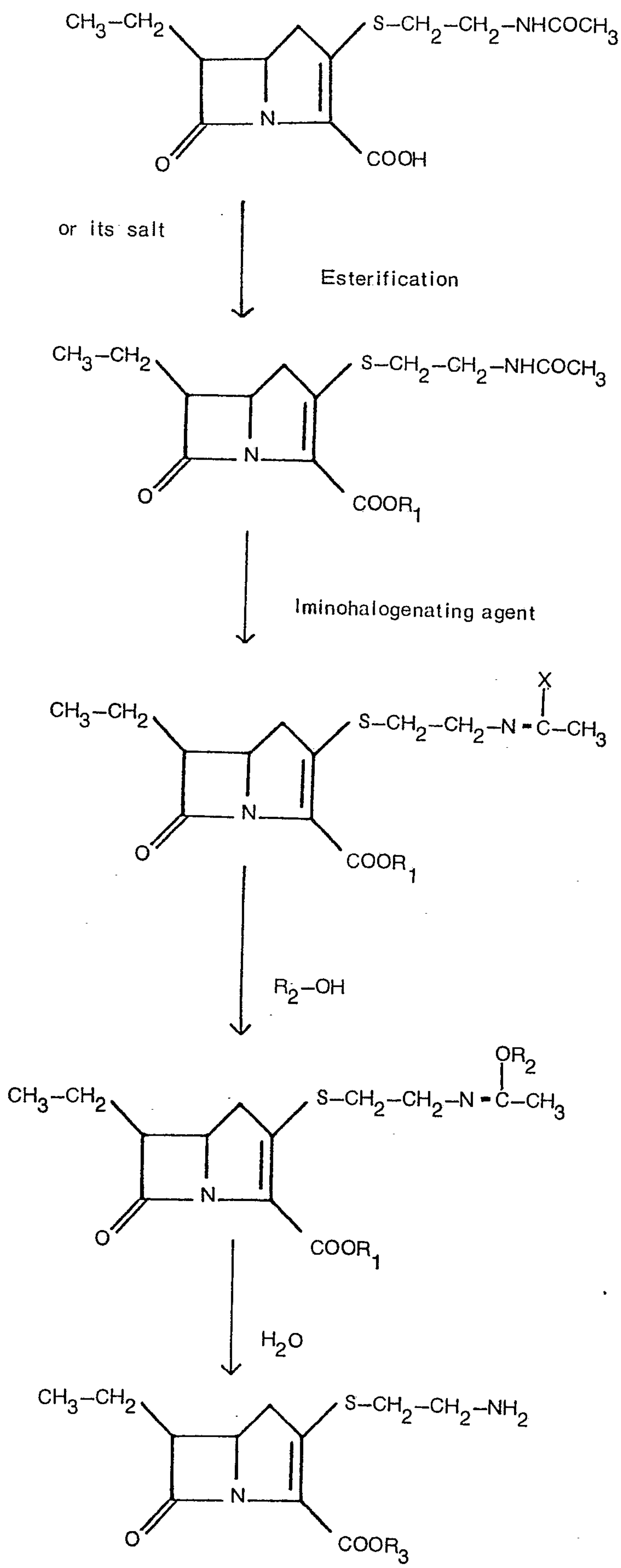
or its salt
Esterification
Iminohalogenating agent
R2–OH
H2O

(IV)

(II)

(V)

(VI)

(I-b)

wherein X represents a halogen atom, $R_3$ represents a hydrogen atom or carboxyl-protecting group, and $R_1$ and $R_2$ are the same as above defined.

The compound of formula (IV) used as a starting material in the above process, that is, the antibiotic PS—5 is a substance which was discovered for the first time by the present inventors.

The antibiotic PS—5 can be produced by a process which comprises cultivating an antibiotic PS—5 producing microorganism in a nutrient medium and isolating antibiotic PS—5.

Representative microorganisms of the above-mentioned antibiotic PS—5 producing strains belong to the genus *Streptomyces*. The most suitable example is a *Streptomyces* strain which was isolated from a soil sample collected near Eiheiji Temple in the Yoshida District of Fukui Prefecture in Japan and given the strain number A271. *Streptomyces* sp.A271 was deposited at the American Type Culture Collection on December 20, 1977, under ATCC 31358, and at the Fermentation Research Institute, Japan, under FERM P—3984 on March 25, 1977, and is available from these depositories.

The antibiotic PS—5 can be produced by inoculating spores of *Streptomyces* sp. A271 into a nutrient medium generally utilized by *Streptomyces*, for example carbohydrates, nitrogen sources and inorganic salts and cultivating it by a method known in the art. For example, it is cultivated aerobically at a temperature of usually 25 to 35°C by a stationary culture, shaking culture or submerged culture method. When a sufficient amount of antibiotic PS—5 has accumulated (usually 20 to 90 hours after the start of fermentation), it is recovered from the fermented product by a known method which has been frequently employed for recovering carboxylic acid-type antibiotics such as penicillin and cephalosporin.

The taxonomical properties of *Streptomyces* sp. A271, a process for the fermentative production of the antibiotic PS—5, isolation and purification thereof, and a process for the production of ester derivatives of the antibiotic PS—5 are fully disclosed in German Appln. P 28 13 922 published as DE—OS 2,813,922 on October 19, 1978 which corresponds to British Appln. 9338/78, in European Application 0,001,567 and in "The Journal of Antibiotics" Vol. 31, No. 5, pg. 480—482 May 1978. A specific example of producing the antibiotic PS—5 and its trityl ester is given in Referential Example to be given hereinbelow.

The compound of formula (II) can be easily prepared by introducing a protective group for the carboxyl function into the antibiotic PS—5 of formula (IV) or a salt thereof by a method known *per se*. For example, it can be prepared by reacting the antibiotic PS—5 or its salt with a compound of the formula

$$R_1Y \qquad \text{(VII)}$$

wherein $R_1$ has the same meaning as above, and Y represents an atom or group that can be cleaved off, or with a lower diazoalkane such as diazomethane.

Y in general formula (VII) may be any kind of atom or group which can be cleaved off when brought into contact with the carboxyl group of the antibiotic PS—5, for example, a halogen atom such as chlorine, bromine, or iodine, a hydroxyl group, a thiol group, a sulfonyloxy group, —COH, and a reactive carbonyloxy group such as $—O—CO—CF_3$. Halogen atoms are particularly preferred.

The reaction of the antibiotic PS—5 with the compound of general formula (VII) or with the lower diazoalkane can be carried out by known methods of esterification. For example, the reaction of the antibiotic PS—5 with the compounds of general formula (VII) or with the lower diazoalkanes can be carried out generally in the absence of a reaction medium. Generally, however, it is carried out in an inert liquid medium. Inert liquid media that can be used include, for example, hydrocarbons such as benzene, toluene, n-hexane or cyclohexane; halogenated hydrocarbons such as chloroform or methylene chloride; amides such as dimethyl formamide or hexamethylphosphoric triamide; dimethyl sufoxide; ethers such as diethyl ether, diisopropyl ether, di n-butyl ether, tetrahydrofuran, or dioxane; esters such as ethyl acetate, or n-butyl acetate; and ketones such as acetone, or methyl ethyl ketone. These solvents can be used singly or as a mixture of two or more.

The reaction temperature is not critical and can be changed broadly depending upon the kind of the compound of general formula (VII), the kind of the lower diazoalkane, the kind of liquid medium, etc. The reaction temperature can be selected from those at which the antibiotic PS—5 is not markedly decomposed. In general, the suitable temperature is not more than 60°C, preferably in the range between 0°C and 40°C, and more preferably in the range between 5°C and room temperature. As required, a reaction promotor such as trimethylamine, triethylamine, pyridine, or dicyclohexylcarbodiimide may be used in the reaction.

Under these conditions, the reaction can be terminated within about 1 to 24 hours, usually 3 to 12 hours.

Carboxyl-protecting groups which can be employed may be any of those which are usually used in the case of antibiotics containing carboxyl groups (for example, penicillanic acid, cephalosporanic acid, clavulanic acid and thienamycin). Particularly suitable are those which can be cleaved off without difficulty under mild hydrolytic conditions. Examples of such a protective group for the carboxyl function and the method of its introduction are shown, for example, in Chapter 5, "Protection of Carboxyl

Group" by E. Haslam of "Protective Groups in Organic Chemistry", J. F. W. McOmie ed., Plenum Press (1973).

Thus, useful carboxyl protecting groups ($R_1$) include alkyl groups, for example, a methyl, ethyl, *n*- or *iso*-propyl, *n*-, *iso*- or tert-butyl, *n*-pentyl, *iso*-amyl, *n*-hexyl, or *n*-octyl group, particularly lower alkyl groups; cycloalkyl groups, for example, a cyclopentyl or cyclohexyl group, preferably cycloalkyl groups having 5 to 10 carbon atoms; and aralkyl groups, for example, a benzyl, phenethyl, benzhydryl, or trityl group, particularly (mono-, di- or tri-phenyl)-lower alkyl groups. These groups may be substituted further by an acyloxy group, for example, an acetoxy, propionyloxy, or benzoyloxy group, particularly a lower alkanoyloxy group; an alkoxy group, for example, a methoxy, ethoxy, *n*- or *iso*-propoxy group, particularly a lower alkoxy group; a nitro group; a halogen atom; or a lower haloalkyl group, for example, a trifluoromethyl group. Examples of the substituted protective group include *p*-nitrobenzyl, *p*-methoxybenzyl, acetoxymethyl, acetoxyethyl, propionyloxymethyl, methoxymethyl, ethoxymethyl, pivaloyloxymethyl, methylthiomethyl, and 2,2,2-trichloroethyl groups.

The term "lower" in this specification is used to show that a group or compound qualified by this term contains up to 10 carbon atoms, preferably up to 6 carbon atoms.

In accordance with this invention, the compound of formula (II) is treated with an iminohalogenating agent to convert it to the iminohalide.

Any type of iminohalogenating agent can be employed for this purpose which can convert an acid-amide group (—NHCO—) to an iminohalide group

$$\left( -N{=}\overset{\overset{\text{halogen}}{|}}{C} \right).$$

Examples of such reagent are phosphorus pentachloride, phosphorus pentabromide, phosphorus trichloride, phosphorus tribromide, phosphorus oxychloride, phosphorus oxybromide, thionyl chloride, thionyl bromide, and phosgene. Phosphorus pentachloride, phosphorus pentabromide, phosphorus oxychloride and phosphorus oxybromide are suitable.

The reaction of the compound of formula (II) with the iminohalogenating agent may usually be carried out in an inert solvent. Examples of the solvent are halogenated hydrocarbons such as carbon tetrachloride, chloroform, or dichloroethane; ethers such as tetrahydrofuran or dioxane; hydrocarbons such as benzene or toluene; and acetonitrile.

In particular, non-protonic organic solvents are suitable. These solvents may contain a small amount of water, but in order to minimize the loss of the iminohalogenating agent, they are preferably dried prior to use.

The ratio of the iminohalogenating agent to the compound of formula (II) is not strictly defined and may be widely changed depending on the kind of the iminohalogenating agent employed. Generally, to one mole of the compound of formula (II), at least 0.9 equivalent, preferably 1 to 20 equivalents, more preferably 1 to 5 equivalents, of the iminohalogenating agent may be used.

The reaction is preferably carried out in the presence of a base. Suitable bases are organic amines, for example, tertiary amines such as triethyl amine or N-alkyl-morpholine, and pyridine. The amount of the base is not critical and may be widely changed depending on the kind of the iminohalogenating agent employed. Generally, to one mole of the iminohalogenating agent, at least 0.9 equivalent, preferably 1 to 5 equivalents, more preferably 2 to 3 equivalents, of the base is used.

The reaction temperature is also not critical. But generally the reaction is carried out preferably at room temperature or at lower temperatures under cooled conditions. Usually, it is performed at a temperature of between room temperature and −70°C, preferably under cooled conditions at 0 to −60°C.

The iminohalogenation reaction can be completed within 30 minutes to 10 hours under these conditions.

In the resulting reaction mixture, an iminohalide compound is formed in which the —$NHCOCH_3$ group of the compound of formula (II) has been changed to

$$-N{=}\overset{\overset{X}{|}}{C}-CH_3$$

group (in which X is a halogen atom).

The resulting iminohalide compound does not need to be isolated, and can be reacted with an alcohol of the formula

$$R_2—OH \qquad (III)$$

wherein $R_2$ represents a hydrocarbon group, either as such or, if desired, after a part of the solvent is distilled off under reduced pressure.

In formula (III), the hydrocarbon group includes a saturated or unsaturated aliphatic group, cycloaliphatic group, aromatic group or araliphatic group which contains up to 15 carbon atoms, preferably up to 10 carbon atoms, more preferably up to 6 carbon atoms, and may contain a hetero atom such as a nitrogen or sulfur atom. Example of suitable hydrocarbon groups are alkyl groups such as a methyl, ethyl, *n*- or *iso*-propyl, *n*-, *iso*- or *tert*-butyl, *n*-pentyl, *iso*-amyl, *n*-hexyl or *n*-octyl group, cycloalkyl groups such as a cyclopentyl or cyclohexyl group; and aralkyl groups such as a benzyl or phenethyl group. Lower alkyl groups, particularly a methyl group, are preferred.

Examples of suitable alcohols of formula (III) thus include methanol, ethanol, *n*- or *iso*-propanol, *n*-, *iso*- or *tert*-butanol; *n*-pentanol, *n*-hexanol, cyclohexanol and benzyl alcohol. Lower alkanols are preferred and methanol is most advantageous.

The amount of the alcohol is not critical. It can be used in a substantially equal mole amount to the imino-halide compound, but preferably a large excess of alcohol is used to make it serve also as a reaction medium. The alcohol used may contain a small amount of water, but in order to minimize the hydrolysis of the iminohalide, it is advantageous to use a substantially anhydrous alcohol.

The iminoetherification with alcohol is preferably carried out at relatively low temperatures to reduce side reactions. Usually the reaction is advantageously performed at below room temperature, preferably under cooled conditions below 0°C, usually between 0 and −78°C, in particular between −20°C and −70°C. The iminoetherification reaction is completed generally within 10 minutes to 5 hours under these conditions. The reaction mixture thus obtained is treated with water without further purification or, if necessary, after at least a part of the alcohol is removed at a low temperature by evaporation under reduced pressure. The desired compound of formula (I) or its derivative protected at the carboxyl group is thus produced.

The treatment with water is generally accomplished by contacting the reaction mixture with a large amount of water. The water may be ordinary water but an aqueous buffer solution at pH 5 to 9 is advantageous. The temperature of the treatment is preferably below room temperature, more preferably between 0 and 20°C.

According to this invention, the procedure above mentioned gives the compound of formula (I) which is protected at the carboxyl group and corresponds to the starting material of formula (II) employed.

Unexpectedly it has been found that when a compound of formula (II) wherein $R_1$ is a trityl group, that is, a trityl ester of the antibiotic PS—5, is employed as the starting material, the trityl group is also cleaved off at some stage of the procedure mentioned above and the compound of formula (I) can be obtained directly, and no particular step for the removal of the carboxyl-protecting group is required. Therefore, when the compound of formula (I) having a free carboxyl function is desired, a trityl group is suitable as the protecting group for the carboxyl function ($R_1$) in formula (II).

As desired, the derivative of formula (I) protected at the carboxyl group obtained by the process above described may be subjected to a treatment for cleaving off the protective group. The cleavage of the protective group for carboxyl function may be performed by a method known *per se.* A typical method is described in the above-mentioned reference, Chapter 5 "Protection of Carboxyl Group" by E. Haslam of "Protective Groups in Organic Chemistry" J. F. W. McOmie ed., Plenum Press (1973).

According to another aspect of this invention, the N-desacetyl derivative of the antibiotic PS—5 of formula (I) is prepared by treating a compound of the following formula

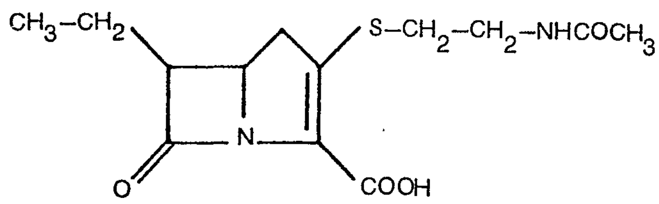

(IV)

that is, the antibiotic PS—5, or a salt thereof with microbial cells or a treated product thereof having the ability to hydrolyze N-acetyl groups.

The salts of the antibiotic PS—5 are, for example, alkali metal salts such as sodium salt, potassium salt and lithium salt; alkaline earth metal salts such as calcium salt and magnesium salt; other metal salts such as aluminium salt; ammonium salts; primary, secondary or tertiary amine salts such as monoethyl amine salt, dimethyl amine salt, trimethyl amine salt, monoethanol amine salt and diethanol amine salt; and organic base salts such as benzathine salt and procaine salt. Alkali metal salts, particularly sodium salt and potassium salt, are preferred.

The biological cleavage of the N-acetyl group from the compound of formula (IV) or salts thereof can be effected by using microbial cells or a treated product thereof having the ability to hydrolyze the N-acetyl group. In this specification and appended claims, the expression "microbial cells having the ability to hydrolyze the N-acetyl group" means microbial cells which have the ability to hydrolyze the

acetamide group of the antibiotic of formula (IV) to a free amino group. According to this invention, microbial cells of any strains having such ability can be used whichever genus or species they may belong to.

The term "treated product of cells" means the microbial cells which have been disrupted by ultrasonic treatment or treatment with a French press and have the ability to hydrolyze the acetamide group of the antibiotic PS—5 to a free amino group. It also includes purified products thereof containing an enzyme (antibiotic PS—5 amidohydrolase).

The "cells having the ability to hydrolyze the N-acetyl group" used in the process of this invention can be easily discovered, for example by the following screening procedure, from microorganisms which have been isolated from the soil by a method known *per se.*

A candidate strain isolated from soil is cultivated in a liquid or solid nutrient medium for 2 to 4 days to grow the cells. The cells are harvested from the culture by refrigeration centrifugation (for example, 3,000 to 8,000 r.p.m. for 5 to 20 minutes), washed in M/100—M/10 phosphate buffer solution, pH 7.0—7.8, and then suspended in the same buffer solution. The suspension is sonically oscillated, for example, with Sonifier UR—200P (Tomy Seiko Co.) for 1 to 5 minutes to disrupt the cells. A cell free-extract (treated cells) is obtained by centrifuging at 10,000 r.p.m. for 10 to 20 minutes under cooling (for example, 0°C).

The cell-free extract is contacted with the antibiotic PS—5 (sodium salt) in M/100—M/10 phosphate buffer solution, pH 7.0—7.8. The contacting may be generally carried out at 20 to 35°C for 1 to 20 hours, but preferably at 25 to 30°C for 1 to 5 hours. Inorganic salts, for example, cobalt chloride may be added to the treating mixture if desired.

The state of progress of a reaction that may take place during the treatment can be monitored by bioassay and high voltage paper electrophoresis, a combination of paper chromatography or thin layer chromatography and bioautography. The size variation of the inhibitory zone at an inherent Rf value and an inherent migration distance in the bioautogram shows the formation of the reaction product, N-desacetyl derivative of the antibiotic PS—5 and the disappearance of the starting material, the antibiotic PS—5. From this change, it can be judged whether the cells or a treated product thereof has the ability to hydrolyze the N-acetyl group of the antibiotic (PS—5).

Bioassay

(1) Cells of *Comamonas terrigena* B—996, cultivated on a nutrient agar overnight, are suspended in a nutrient broth to give a seed culture having an absorbance at 610 mm, ascribable to th cells, of 0.04. A melted agar medium consisting of 0.8% Kyokuto powdered bouillon (Kyokuto Seiyaku Kogyo) and 1% Bacto agar (Difco Labs.) is inoculated with 1% of the seed culture. Seven ml portions of the agar medium are poured into petri dishes with a diameter of 9 cm and solidified to prepare a *Comamonas* assay plate.

(2) *Staphylococcus aureus* FDA209P is cultivated overnight with shaking in a nutrient broth. The culture is diluted to 50 times with a nutrient broth to obtain a seed culture. A melted agar medium consisting of 1% Kyokuto powdered bouillon (Kyokuto Seiyaku Kogyo) and 1% Bacto agar (Difco Labs.) is inoculated with 1% of the seed culture. Seven ml portions of the agar medium are poured into petri dishes with a diameter of 9 cm and solidified to prepare a *Staphylococcus* assay plate.

(3) A pulp disc with a diameter of 8 mm is soaked in a sample solution and put on the assay plate mentioned above after removing an excess of the sample solution soaked by placing on filter paper. The plate is incubated at 35°C for 20 hours. The diameter of the inhibitory zone is measured. The concentration of the test compound in the sample solution is calculated by comparing the diameter with that of the standard solution.

Bioautography

In the above-mentioned bioassay, a 32 cm long and 24 cm wide tray is used in place of the 9 cm-diameter petri dish. One hundred ml of the agar medium inoculated with the seed culture is spread in the tray and solidified to prepare a large assay plate.

The paper chromatogram of the sample is placed on the agar surface of the large assay plate for 15 minutes. After the paper has been removed, the plate is incubated at 35°C for 20 hours. The Rf value of the paper chromatogram is calculated from the position of the inhibitory zone. The amount of the assay sample can be roughly determined from the size of the inhibitory zone.

In the case of a thin layer chromatogram, the chromatogram is placed on the assay plate with a sheet of thin paper put between the chromatogram and the assay plate in such a manner that the developed thin film side contacts the surface of the agar medium. 15 minutes later the chromatogram is taken off from the surface of assay plate and then the assay plate is incubated overnight. The Rf value is calculated and the amount of sample is roughly determined in the same manner as mentioned above.

Studies by the present inventors have shown that *Protaminobacter ruber* NRRL B—8143 (FERM No. 3794), — deposited November 11, 1976 — already known as an N-acetylthienamycin amidohydrolase-producing strain (Japanese Laid-Open Platent Publication No. 65295/77), is a suitable organism having the ability to hydrolyze the N-acetyl group.

Cultivation of such microorganisms can be carried out by a method known *per se* (for example,

the method described in Japanese Laid-Open Patent Publication No. 65295/77 described above). The cells may usually be harvested by filtration, centrifuging and the like. Generally high-speed centrifuging is suitable for the recovery of bacterial cells. Separation of the cells from solid matter in the culture broth can be carried out by centrifugation based on the difference in their specific gravities. The recovered cells may be used without any treatment in the process of this invention but they may also be used as treated cells prepared by various methods including disruption of cells by ultrasonic or French press treatment; removal of the cell debris by centrifuging for preparation of a cell-free extract; Streptomycin treatment for removal of nucleic acid; salting-out fractional precipitation, for example, ammonium sulfate fractionation, for obtaining a concentrated enzyme preparation containing the enzyme capable of hydrolyzing the N-acetamide group of the antibiotic PS—5 of formula (IV) (antibiotic PS—5 amidohydrolase). A treated cell preparation obtained by column chromatography on DEAE cellulose (Brown) or DEAE Sephadex (Pharmacia AB) may also be used.

The reaction of the antibiotic PS—5 or salts thereof with the cells or treated cells which are capable of hydrolyzing the acetamide group to an amino group can be carried out by employing a known method, for example, the process described in Japanese Laid-Open Patent Publication No. 65295/77. More specifically, the reaction can be accomplished under the following conditions.

The concentration of the antibiotic PS—5 or a salt thereof in the reaction mixture may be in the range between 10 and 50,000 $\mu$g/ml, preferably 20 and 1,000 $\mu$g/ml. The amount of the cells or treated cells used depends on the content of the hydrolase. Namely, when one unit of the enzyme is defined as the amount required for the hydrolysis of one micromole of the antibiotic PS—5 per hour, it is advantageous that the cells or treated cells are added so that the concentration during the reaction is in the range between 0.001 and 100 units/ml, preferably 0.05 and 1.0 units/ml. Generally, the reaction may be carried out in an aqueous buffer solution, the pH of the reaction mixture being usually 6.5—9.5, preferably 7.0—8.0. A trace amount of inorganic salts such as cobalt chloride may be added to the reaction mixture if desired. The reaction is carried out at a temperature ranging between 5° and 55°C, preferably 20° and 35°C, and may be continued usually for 10 minutes to 20 hours, preferably 30 minutes to 3 hours.

Paper chromatography and high voltage paper electrophoresis combined with the bioassay and bioautography described above can show the formation of the reaction product (N-desacetyl derivative) and the decomposition of the antibiotic PS—5 or salts thereof.

The N-desacetyl derivative of the antibiotic PS—5 thus produced by the chemical or biological process described above may be recovered by various methods known *per se.* In particular methods often employed for the isolation and purification of water-soluble amphoteric antibiotics are advantageously applied. For example, a combination of adsorption on active carbon, Amberlite XAD—2 (Rohm and Haas Co.) or Diaion HP—20 (Mitsubishi Chemical Industries, Ltd.) and elution with methanol/water or acetone/water; adsorption and elution with ion exchange resins such as Dowex 1x2 (Dow Chemicals Co.), Dowex 50x2 (Dow Chemical Co.) or QAE—Sephadex A—25 (Pharmacia AB); gel filtration with Sephadex G—10 (Pharmacia AB) or Biogel P—2 (BioRad Labs); column or thin layer chromatography with cellulose, Avicel SF (American Viscose Co.), DEAE cellulose Whatman DE—32 (Whatman Co.), DEAE Sephadex A—25 (Pharmacia AB), silica gel or alumina; forced precipitation with solvents such as acetone; and lyophilization. They may be employed independently or in combination, and repeatedly if necessary.

The behaviour of the N-desacetyl derivative during the purification and recovering step can be quantitatively analyzed by the bioassay and bioautography described above.

The N-desacetyl derivative of the antibiotic PS—5 is thus obtained. The N-desacetyl derivative has the following properties.

A) Solubility

The N-desacetyl derivative is easily soluble in water but practically insoluble in acetone, ethyl acetate and benzene.

B) Paper chromatography

Descending chromatography of the N-desacetyl derivative on filter paper Toyo Roshi No. 50 and the following solvent system gives the Rf value described below.

Acetonitrile/Tris/EDTA[1]) RF = 0.22
$Rf_{PS-5}$ = 0.64[2])

NOTE: 1) A mixed solvent system consisting of acetonitrile 120 ml, M/10 tris-(hydroxymethyl) aminomethane-hydrochloric acid buffer, pH 7.5, 30 ml and M/10 aqueous tetrasodium ethylenediamine tetraacetate solution, pH 7.5, 1.0 ml.

2) Relative Rf value of the N-desacetyl derivative to PS—5 when the migration distance of PS—5 is 1.0:

$$Rf_{PS-5} = \frac{\text{Rf value of the N-desacetyl derivative}}{\text{Rf value of the antibiotic PS—5}}$$

C) High voltage paper electrophoresis

The N-desacetyl derivative of the antibiotic PS—5 shows the following migration in a high voltage paper electrophoresis with a buffer solution of the pH value described below on Whatman No. 1 filter paper (Whatman Co.) with High Voltage Paper Electrophoresis Apparatus (Savant Instruments Co., High voltage power supply HV3000A, Migration vessel FP18A).

The compound migrates through a distance of at least 0.5 mm, usually in the range between 3 and 30 mm, toward the cathode in the buffer solution, pH 8.6, consisting of barbital 3.3 g, sodium barbital 25.5 g and water 3000 ml with a 30 minute-charge at 42 V/cm.

D) Behaviour against $\beta$-lactamase

The N-desacetyl derivative is partially inactivated by $\beta$-lactamase from *Proteus vulgaris* and *Citrobacter freundii.*

E) Distinction among acid, neutral and base

The N-desacetyl derivative is an amphoteric compound that has one carboxyl and one amino group in the molecule.

F) Ultraviolet absorption spectrum

The N-desacetyl derivative shows the following characteristic absorption maximum in the ultraviolet absorption spectrum measured for its solution in 0.05M phosphate buffer, pH 7.2.

$\lambda$ max = approx. 293 nm

G) Color reactions

Iodine-chloroplatinic acid reaction: positive Ninhydrin reaction: positive

H) Infrared absorption spectrum

The infrared absorption spectrum of the N-desacetyl derivative in a KBr tablet has characteristic absorption maxima at the following wave lengths:

A sharp peak at 1765 $cm^{-1}$ (—CO— of $\beta$-lactam ring

A broad peak at 1600 $cm^{-1}$ (—$COO^-$ of carboxylate)

I) Proton nuclear magnetic resonance spectrum

In a 100 MHz proton nuclear magnetic resonance spectrum in $D_2O$, the N-desacetyl derivative of the invention shows the following characteristic signals.

(i) Triplet having a center at 1.06 ppm (J = 7.8 Hz) ($CH_3$—$CH_2$—)

(ii) Multiplet at 1.70—2.00 ppm ($CH_3$—$CH_2$—)

(iii) Multiplet at 3.12—3.32 ppm (—$CH_2$—, —$\overset{|}{C}H$—)

The above NMR spectrum does not at all show a sharp singlet ascribable to the acetyl methyl group which is observed at 2 ppm in the NMR spectrum of the antibiotic PS—5.

J) Antimicrobial spectrum

The N-desacetyl derivative according to the present invention shows a high antimicrobial activity on staphylococcal bacteria.

K) Activity in vivo

The derivative showed a remarkable therapeutic effect on mice infected with pathogenic Gram-positive bacteria.

L) Toxicity

No acute toxicity was observed in mice to which the derivative was administered intraperitoneally at a dose of 500 mg/kg.

From the chemical structure of the starting material, the antibiotic PS—5, the reactions for formation of the product, the physicochemical and the biological properties mentioned above, the N-desacetyl derivative of the antibiotic PS—5 provided by the present invention is determined to be the compound having the following structural formula (I).

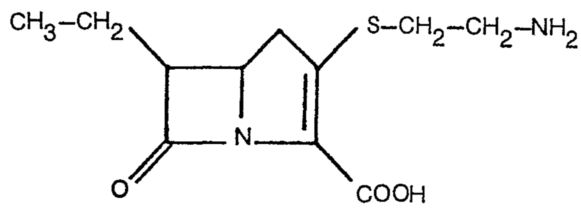

(I)

The compound of formula (I) has a high antimicrobial activity as mentioned above and can be effectively used as an active component of an antimicrobial agent for prevention, therapy and or treatment of infections with Gram-positive and Gram-negative bacteria not only in man but also in other animals including mammals, poultry or fish.

The compound of formula (I) in accordance with the present invention may be administered orally, topically or parenterally (intravenously, intramuscularly, intraperitoneally,) and may be used in any one of a variety of usual pharmaceutical preparations according to the route of administration. For example, the N-desacetyl derivative of the antibiotic PS—5 of this invention may be prepared together with a pharmacologically acceptable carrier, diluent or the like in solid forms (for example, tablets, capsules, powders, granules, dragees, troches, powder sprays, suppositories,), semi-solid forms (for example, ointments, creams, semi-solid capsules,), or liquid forms (for example, liquid solutions, emulsions, suspensions, lotions, syrups, solution for injection, liquid sprays,).

The unit dose preparation containing the compound of formula (I) may contain generally 0.1 to 99 weight %, preferably 10 to 60 weight % of the active component in any of liquid, semi-solid and solid forms.

Representative carriers, fillers, diluents, or the like which can be used in these preparations and the methods of formulation are described below in detail.

Tablets and capsules for oral administration may be in unit dose preparation form, and may contain binding agents, for example, syrup, acacia, gelatin, sorbitol, tragacanth, polyvinylpyrrolidone or the like; fillers, for example, lactose, sucrose, starch, calcium phosphate, sorbitol or glycine; lubricants, for example, magnesium stearate, talc, polyethylene glycol or silica; disintegrants, for example, potato starch; or wetting agents such as sodium lauryl sulfate.

The tablets may be coated according to method well known in the art.

Liquid preparations for oral uses may be in the form of oily or aqueous suspensions, solutions, emulsions, syrups, or may be provided as dry products that can be reconstituted with water or other suitable vehicles before use. The liquid preparations for oral uses may contain the following pharmaceutically acceptable ingredients: suspending agents (for example, methyl cellulose, sorbitol syrup, sugar syrup, hydroxyethyl cellulose, gelatin, carboxymethyl cellulose, aluminum stearate gel, hydrogenated edible fats and oils); emulsifying agents (for example, acacia, lecithin, sorbitan monooleate); non-aqueous vehicles (for example, ethyl alcohol, propylene glycol, oily esters, fractionated coconut oil, almond oil); preservatives (for example, methyl p-hydroxy-benzoate, propyl p-hydroxybenzoate, sorbic acid).

Suppositories may contain conventional suppository bases like cocoa butter and various glycerides.

Preparations for injection may be prepared in unit dose form in ampoules or in multidose containers with a preservative. They may be in the form of suspensions, solutions and emulsions in oily or aqueous vehicles, and if necessary, may contain formulatory agents such as suspending agents, dispersing agents and stabilizing agents. Alternatively, the antibiotic of this invention may be prepared in the powder form reconstitutable in pyrogen-free, sterilized water before use.

Compositions containing the compound of formula (I) may be provided in various forms suitable for absorption through the mucous membrane of the nose and throat and the bronchial tissues. The form of powder or liquid sprays, inhalants, trouches or throat coats, will be advantageous for the above purposes. For treatment of the ears and eyes, the antibiotic of this invention may be prepared as individual capsules, as drops, in liquid or semi-solid form. In addition, for topical applications, it may be presented as formulations in hydrophilic or hydrophobic bases such as powders, lotions, creams, or ointments.

If desired, in addition to a carrier, the preparations described above may contain other ingredients, for example, preservatives, antioxidants, lubricants, viscosity agents, flavoring agents, suspending agents, binders or stabilizing agents.

When the compound of formula (I) according to this invention is intended for veterinary uses such as treatment of infections in pigs, cows, sheep or chickens, the formulations may be presented as intramammary preparations in long-acting or quick-releasing bases, for instance, or as concentrated feed additives by a known method.

The above-described pharmaceutical preparations in accordance with this invention may contain the compound of formula (I) as the sole active ingredient or in combination with other therapeutically effective ingredients.

The compound of formula (I) may be combined with known β-lactam antibiotics in pharmaceutical compositions. As suitable examples of such β-lactam antibiotics can be listed Penicillin antibiotics such as Benzylpenicillin, Phenoxymethylpenicillin, Carbenicillin, Ampicillin and Amoxicillin; and Cephalosporin antibiotics such as Cephaloridine, Cephalothin, Cefazolin, Cephalaxin, Cefoxitin, Cephacetrile, Cephamandole, Cephapicin, Cephradine and Cephaloglycin.

When the compound of formula (I) in accordance with this invention is combined with one or more members of the above-listed β-lactam antibiotics, the combining ratio of the compound of this invention to the known β-lactam antibiotics is not critical, and may vary in a wide range. But, from the practical viewpoint, it is advisable to use the quantitative ratio of the compound of formula (I) to the known β-lactam antibiotics in the range of 20:1 to 1:150; and preferably 10:1 to 1:100.

In the treatment of bacterial infections in man, the dose of the compound of formula (I) according to this invention can be varied depending on the object to be treated, the body weight, the type, severity and symptom of infections, the mode and number of administration. For usual oral or parenteral administration, it is profitable to use a daily dose in the range of 0.05 mg/kg to 500 mg/kg, preferably 0.5 mg/kg to 200 mg/kg in a single or divided administration. It will be well understood that a responsible physician can choose the dose beyond the above-recommended range, depending on the individual conditions of a patient to be treated.

The compound of formula (I) according to this invention not only can be used as pharmaceutical preparations described above but also may be directly added to animal feeds or used for producing animal feed additive concentrates. In addition, it may be utilized as the active ingredients for food preservatives or disinfectants.

The following Examples illustrate this invention.

Antibiotic PS—5 and trityl ester thereof used in the following examples were prepared according to the referential examples.

Referential Example 1: Sodium salt of PS—5

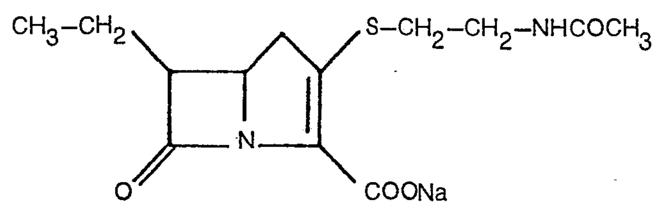

A seed medium (SE—4, 100 ml) of the following composition was poured into a 500 ml=Erlenmeyer flask and sterilized at 120°C for 15 minutes. A spore suspension was prepared by adding 10 ml of 0.02% Tween 80 (Atlas Co., surfactant) aqueous solution in a slant culture tube of *Streptomyces* sp. A271 that had already been in good sporulation and stirring lightly. The seed culture medium was inoculated with 1 ml of the spore suspension and incubated on a rotary shaking machine (200 r.p.m., 7 cm stroke) at 28°C for 48 hours.

Fifteen liters of SE—4 medium in a 30 liter-jar fermentor was inoculated with 100 ml of the seed obtained by the flask culture and incubated at 28°C for 24 hours agitation at 200 r.p.m. and aeration at 7.5 l/min to prepare a seed culture.

Two-200 liter stainless steel fermentors were filled each with 100 liters of the ML—19B medium and sterilized. One liter of the seed culture was inoculated, and cultivation was performed for 72 hours under aeration and agitation.

*Seed culture medium (SE—4)*

| | |
|---|---|
| Beef extract (Difco Laboratories) | 0.3% (w/v) |
| Bacto-tryptone (Difco Laboratories) | 0.5% |
| Glucose | 0.1 |
| Soluble starch | 2.4 |
| Yeast extract | 0.5 |
| Calcium carbonate | 0.4 |
| Defatted soybean meal | 0.5 |
| pH 7.5 prior to sterilization | |

*ML—19 medium*

| | |
|---|---|
| Glycerin | 4.0% (w/v) |
| Peptone | 0.5 |
| Glucose | 0.2 |
| Potato starch | 0.2 |
| Defatted soybean meal | 0.5 |
| Dry yeast | 0.5 |
| NaCl | 0.5 |
| $CaCO_3$ | 0.2 |

pH 6.4 prior to sterilization

The contents of the two fermentors were combined, mixed with 5% (w/v) Topco Perlite, No. 34 (Topco Perlite Kogyo K.K.) and filtered by a filter press to give 160 liters of a broth filtrate. The antibacterial potency of this filtrate was found to be 235 CCU/ml (The potency of the pure sodium salt of PS—5 is 21,000 CCU/mg.).

This broth filtrate was passed through an ion exchange resin column (DIAION PA—306, Mitsubishi Chemical Industries, Ltd.; 10 × 52 cm; 4.5 liters in wet volume). Then the effluent was adsorbed on a DIAION HP—20 (Mitsubishi Chemical Industries, Ltd.) column (14 × 97 cm; 15 liters). The active material was eluted with 75% methanol. The collected active eluate (2 liters; 9,854 CCU/ml) was diluted three times with 4 liters of water and applied to a 2 liter-column (5.5 × 84 cm) of DIAION PA—306S (Mitsubishi Chemical Industries, Ltd.). After washing with 500 ml of water, the active substance was eluted with an 8-liter linear sodium chloride gradient from 0 to 3% and collected in 200 ml fractions. Active fractions from No. 17 to No. 31 were combined to obtain 2.8 liters of active eluate (4,120 CCU/ml).

The eluate was then charged on a 1-liter DIAION HP—20 column (4.5 × 63 cm). Elution was carried out with a 3-liter linear gradient of acetone from 0 to 25%. The volume of each fraction was 30 ml. The active eluate (390 ml; 27,200 CCU/ml) was recovered by combining antimicrobially active fractions from No. 54 to No. 66.

After acetone was removed by distillation under reduced pressure, the DIAION HP—20 eluate was passed through a 200 ml column of QAE-Sephadex A—25 (Pharmacia Fine Chemicals AB) (2.5 × 41 cm). The antimicrobial activity was collected in 10 ml fractions by elution with a 2-liter linear sodium chloride gradient (0—1.5%). Fractions Nos. 68 to 81 were combined as the active eluate (140 ml; 42,120 CCU/ml).

This QAE—Sephadex A—25 eluate was carefully adjusted to pH 8.3 with 1% NaOH and charged into a 200 ml DIAION HP—20AG (Mitsubishi Chemical Industries, Ltd.; 2.5 × 41 cm) column. Linear gradient elution was carried out with 1 liter of aqueous acetone from 0 to 10%. The volume of each fraction was 10 ml. Fractions Nos. 48 to 53 were combined to give 60 ml of active eluate (45,000 CCU/ml). Freeze-drying provided 249 mg of yellow powder (8,000 CCU/mg).

For further purification, 150 mg of the yellow powder was dissolved in a small amount of 0.01 M sodium phosphate buffer (pH 8.0) and passed through a 130 ml column (1.5 × 73 cm) of Sephadex G—10 (Pharmacia Fine Chemicals AB). It was developed with 0.01 M, pH 8.0, sodium phosphate buffer, the eluate being fractionated in a 2-ml amount. Thirty-six milliliters of the active eluate was obtained from fractions Nos. 38 to 55 (65,800 CCU/ml).

The Sephadex G—10 eluate was then subjected to QAE—Sephadex A—25 (Pharmacia Fine Chemicals AB) column chromatography (100 ml; 2.0 × 32 cm) followed by linear gradient elution with 1 liter of sodium chloride solution from 0 to 1.5%. The fraction volume was 10 ml. Five active fractions from Nos. 48 to 52 (50 ml; 22,000 CCU/ml) were combined as the active eluate.

This active eluate was carefully adjusted to pH 8.3 with 1% NaOH and charged into a 50-ml DIAION HP—20AG (Mitsubishi Chemical Industries, Ltd.) column (1.2 × 44 cm). Active material was recovered in 5-ml fractions by linear gradient elution with 400 ml of aqueous acetone from 0 to 10%. Active fractions Nos. 39 to 41 were combined and lyophilized to yield 51 mg of PS—5 sodium salt as a white powder (21,000 CCU/mg).

The resulting product showed a purity of about 95%. Its ultraviolet absorption spectrum, infrared absorption spectrum, proton nuclear magnetic resonance spectrum and $C^{13}$-magnetic resonance

spectrum and the high resolution mass spectrum of the methyl ester of the product led to the confirmation that this product is antibiotic PS—5 having the following formula

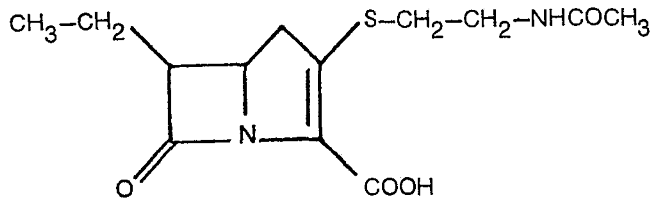

The ultraviolet absorption spectrum, infrared absorption spectrum, proton nuclear magnetic resonance spectrum and high resolution mass spectrum data are shown below.

1) *Ultraviolet absorption spectrum*

Sixty micrograms of antibiotic PS—5 sodium salt was dissolved in 3.0 ml of water and measured in a Hitachi Recording Spectrophotometer Model EPS—3T (Hitachi Ltd.).

The following characteristic values were calculated:

$$\lambda^{H_2O}_{min} = 246 \text{ nm } (E^{1\%}_{1\,cm} = 82.0)$$

$$\lambda^{H_2O}_{max} = 301 \text{ nm } (E^{1\%}_{1\,cm} = 267.5)$$

To an aqueous solution of this preparation (21,000 CCU/mg) in distilled water, a hydroxylamine solution (pH 7.5) was added to make a reaction mixture containing 21.3 $\mu$g/ml of antibiotic PS—5 sodium salt and 10 mM of hydroxylamine. After 30 minutes at 22°C, the reaction mixture lost about 94% of the initial optical density at 301 nm.

2) *Infrared absorption spectrum*

The infrared absorption spectrum of antibiotic PS—5 sodium salt in KBr was recorded on a Hitachi Infrared Spectrophotometer Model 215 (Hitachi Ltd.). The following characteristic absorption maxima were located at the indicated wave numbers:

(i) ca. 1750 $cm^{-1}$ (—CO— in the $\beta$-lactam ring)
(ii) ca. 1650 $cm^{-1}$ (—CO— in the amide bond)
(iii) ca. 1640—1540 $cm^{-1}$ ($—COO^{\ominus}$)

3) *Proton nuclear magnetic resonance spectrum*

The 100 MHz proton magnetic resonance spectrum of antibiotic PS—5 sodium salt in heavy water was recorded in a JEOL NMR spectrometer JNM PS—100 (Japan Electron Optics Laboratory Co., Ltd.). The following characteristic signals were confirmed:

(i) triplet that has a center around 1.06 ppm (j = ca. 7.5 Hz) ($C\mathit{H}_3—CH_2—$)
(ii) multiplet in the region of 1.72—2.00 ppm ($CH_3—C\mathit{H}_2—$)
(iii) sharp singlet around 2.05 ppm ($C\mathit{H}_3—CO—$)
(iv) multiplets in the region of 2.88—3.58 ppm ($—C\mathit{H}_2—, —\overset{|}{C\mathit{H}}—$)
(v) multiplet in the region of 3.0—4.20 ppm 4.04 ppm (1H, doublet, triplet J = 3.0, 9.2 Hz)

(—CH)
|
N

This coupling constant represents 5,6-trans-stereo configuration of PS—5.

4) *Molecular weight*

Approximately 298 (calculated from the results) of high resolution mass spectrometry for the methyl ester of antibiotic PS—5).

Referential Example 2

Trityl ester of antibiotic PS—5

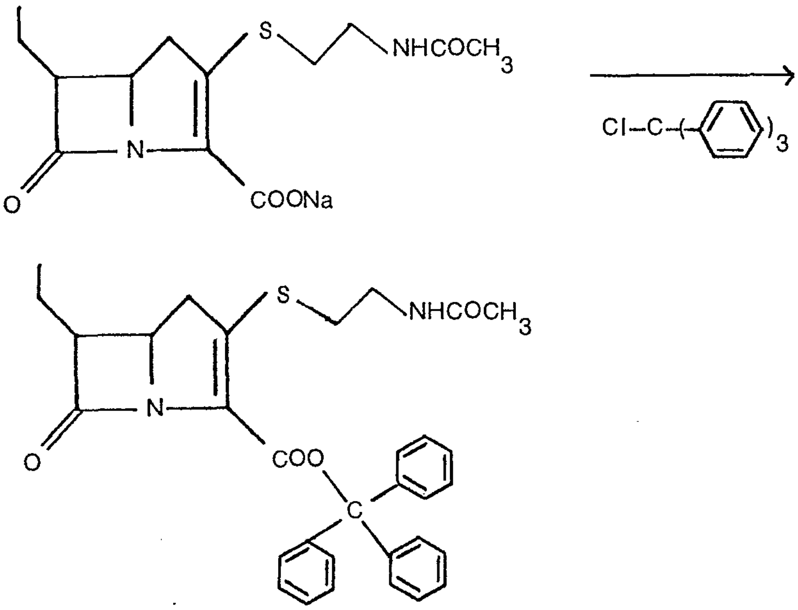

The sodium salt of antibiotic PS—5 (50 mg) obtained by the method of Referential Example 1 was dissolved in dry dimethylformamide (5 ml), and triethylamine (0.03 ml) was added. With stirring, triphenyl methyl chloride (80 mg) was added. The mixture was stirred at 5°C for 12 hours, and then diluted with benzene (50 ml). The solution was washed with 50 ml of 0.1M phosphate buffer at pH 6.8 and 50 ml of water, and dried over anhydrous sodium sulfate. The solvent was distilled off. The resulting gum-like substance was chromatographed on a column, 2.5 cm in diameter and 50 cm in length, packed with Bio-Beads S—X3 (porous styrene-divinylbenzene copolymer beads for gel permeation, molecular weight exclusion limited: 2000, produced by BIO—RAD Laboratories) using benzene as a developing solvent. The resulting active fractions were combined, and concentrated under reduced pressure to dryness. The dried product was dissolved in 0.5 ml of acetone, passed through a Sephadex LH—20 (Pharmacia) column (1.2 x 96.0 cm) previously swollen with acetone, and developed with acetone. The resulting active fractions were combined, and concentrated under reduced pressure to dryness thereby to afford a white powder. Recrystallization from benzene-hexane afforded 21 mg (10,800 CCU/mg) of the trityl ester of antibiotic PS—5 as a white powder.

The structure of this product was identified by elementary analysis, ultraviolet absorption spectrum, infrared absorption spectrum, proton nuclear magnetic resonance spectrum, $C^{13}$-magnetic resonance spectrum, and high resolution mass spectrum. The results obtained with the ultraviolet absorption spectrum, infrared absorption spectrum, proton nuclear magnetic resonance spectrum and elemental analysis are shown below.

1) *Ultraviolet absorption spectrum*

The ultraviolet absorption spectrum of the tritylation product of antibiotic PS—5 of the present invention was recorded in a Hitachi Recording Spectrophotmeter Model EPS—3T (Hitachi Ltd.) at a concentration of 128 $\mu$g/3 ml of methanol.

$$\lambda^{MeOH}_{max} = 315.5 \text{ nm } (E^{1\%}_{1\,cm} = 156)$$

2) *Infrared absorption spectrum*

The infrared absorption spectrum of the tritylation product of antibiotic PS—5 (4.5 mg) in 0.6 ml of chloroform was recorded in a Hitachi Infrared Spectrophotometer Model 215 (Hitachi Ltd.). The characteristic absorption maxima were observed at the following number:

3430

3100—3000 (C—H of the phenyl group)

2990

2950

1770 (C=O of the $\beta$-lactam ring)

1695 (—CO— of the amide bond)

1665 (—CO—O— of the ester bond)

1445

1340

1275

$1130^{-1}$

3) *Proton nuclear magnetic resonance spectrum*

The 100 MHz proton magnetic resonance spectrum of antibiotic PS—5 trityl ester was recorded with 4.5 mg of the trityl ester in 0.3 ml of deuterochloroform, utilizing a JEOL NMR spectrometer JNM PS—100 (Japan Electron Optics Laboratory Co., Ltd.). The most characteristic signals are the following:

triplet around 1.10 ppm (J = ca. 7.0 Hz)
singlet at 1.86 ppm
multiplet in the region of 7.10—7.56 ppm
(protons in the benzene ring of the trityl group)

4) *Elementary analysis*

Three and a half milligrams of the tritylation product of antibiotic PS—5 were dried at room temperature for 4 hours at a reduced pressure of $1 \times 10^{-2}$ mmHg and subjected to the elementary analysis to give the following analysis data:

Found:

| | |
|---|---|
| C | 61.89% |
| H | 5.78% |
| N | 4.48% |
| S | 4.62% |

Example 1

Five-hundred mg of trityl ester of the antibiotics PS—5 prepared according to Referential Example 2 was dissolved in 20 mg of tetrahydrofuran. The solution was cooled with acetone-dry ice and 2 ml of triethylamine was added. Then 1 g of phosphorus pentachloride was added with stirring. After stirring for 2 hours at the same temperature, 5 ml of methanol was added. The mixture was stirred for further 1 hour and poured into a mixture of 200 ml of 0.1 M phosphate buffer solution, pH 6.8 and 200 ml of methanol to form a homogeneous solution. Methanol was removed by evaporation under reduced pressure at a tempeature below 10°C and the residual aqueous solution was washed with 100 ml of benzene, and then the pH of the solution was adjusted to 7.2. The solution was applied to a QAE-Sephadex A—25 ($\phi$ 1.5 cm × 40 cm. Pharmacia AB) and the column was washed with 50 ml of water. The effluent and the washings were combined and 2.5 g of sodium chloride was added. The solution was adsorbed on a DIAION HP—20AG column ($\phi$ 1.5 cm × 40 cm, Mitsubishi Chemical Industries, Ltd.) and eluted with a gradient from 0% to 10% aqueous acetone in a total volume of 600 ml. The eluate was fractionated in a 6 ml-volume each. Lyophilization of the combined fraction from No. 15 to No. 22 provided 24 mg of N-desacetyl derivative of the antibiotic PS—5 in white powder.

The obtained N-desacetyl derivative showed the following physicochemical and biological properties.

A) Solubility

Soluble in water and practically insoluble in acetone, ethyl acetate and benzene.

B) Paper chromatography

The following Rf value was shown by a descending method on Toyo Filter Paper No. 50 (Toyo Roshi Co.) with the following solvent system:

Acetonitrile/Tris/EDTA[1])
Rf = 0.22
$Rf_{PS-5}$ s 0.64[2])

NOTE: 1) A mixed solvent system consisting of acetonitrile 120 ml, M/10 tris-(hydroxymethyl)-aminomethane-hydrochloric acid buffer, pH 7.5 30 ml and M/10 aqueous tetrasodium ethylene-diamine tetraacetate solution, pH 7.5 1.0 ml.

2) Relative Rf value of the N-desacetyl derivative to the antibiotic PS—5 when the migration distance of PS—5 is 1.0:

$$Rf_{PS-5} = \frac{\text{Rf value of the N-desacetyl derivative}}{\text{Rf value of the antibiotic PS—5}}$$

C) High voltage paper electrophoresis

The derivative showed the following migration in the electrophoresis with the buffer solution of the pH value described below on Whatman No. 1 filter paper (Whatman Co.) with High Voltage Paper Electrophoresis Apparatus (Savant Instruments Co., High voltage power supply HV3000A, Higration vessel FP18A):

The derivative migrates through a distance of 5 mm toward a cathode in the buffer solution, pH 8.6, consisting of barbital 3.3 g, sodium barbital 25.5 g and water 3000 ml with a 30 minutes-charge at 42 V/cm.

D) Ultraviolet absorption spectrum

The following characteristic absorption maximum was shown when the ultraviolet absorption spectrum was measured in the solution in 0.05 M phosphate buffer, pH 7.2. The recorded chart is shown in the attached Figure 1.

$$\lambda max = 293 \text{ nm}$$

E) Color reactions

Iodine-chloroplatinic reaction: positive

Ninhydrin reaction: positive

F) Infrared absorption spectrum

The attached Figure 2 shows the infrared absorption spectrum of the N-desacetyl derivative in KBr tablet recorded on a Hitachi Infrared Spectrophotometer Type 215 (Hitachi Ltd.). The following characteristic absorption maxima were located at the indicated wave numbers:

(i) A sharp peak at 1765 $cm^{-1}$

(ii) A broad peak at about 1600 $cm^{-1}$

G) Proton nuclear magnetic resonance spectrum

The 100 MHz proton nuclear magnetic resonance spectrum of the product measured in $D_2O$ using JNM PS—100 (made by Nippon Denshi) showed the following characteristic signals.

(i) Triplet having a center at about 1.06 ppm (J = about 7.8 Hz) ($CH_3$—$CH_2$—)

(ii) Multiplet at about 1.70—2.00 ppm ($CH_3$—$CH_2$—),

(iii) Multiplet at about 3.12—3.32 ppm (—$CH_2$—, —CH—

The above spectrum did not at all show a sharp singlet ascribable to the acetyl methyl group which is observed at about 2 ppm in the NMR spectrum of antibiotic. The obtained n-desacetyl derivative of PS—5 is believed to have 5,6-trans configuration of the formula (I) in view of the fact that the starting antibiotic PS—5 has 5,6-trans configuration.

H) Antimicrobial spectrum

The minimum inhibitory concentration was determined on various pathogenic microorganisms including a resistant strain to the N-desacetyl derivative by the broth dilution method with a brain heart infusion medium "Eiken" (Eiken Kagaku Co.). Sample solutions to be tested were prepared in a serial dilution with the medium, which were inoculated with the test microorganisms at the final cell count of about 1 x $10^5$/ml and incubated at 35°C. After 20 hours the microbial growth was examined. The minimum inhibitory concentration was determined as the lowest concentration of the sample at which no growth of the corresponding test microorganism was observed.

Table 1 gives the results of the derivative together with those of the known antibiotic Cefoxitin as the control.

TABLE 1

| Microorganism | Minimum inhibitory concentration (μg/ml) | |
|---|---|---|
| | N-Desacetyl derivative | Cefoxitin |
| *Staphylococcus aureus* FDA 209P | 0.008 | 1.56 |
| *Staphylococcus aureus* Russel | 0.063 | 3.13 |
| *Klebsiella pneumoniae* K–2 | 5.0 | 12.5 |

I) Activity in vivo

The N-desacetyl derivative was prepared on a large scale in a similar manner to the above-described and the activity in vivo was determined in mice inoculated with 5 × $10^5$ cells/mouse of *Staphylococcus aureus* Smith intraperitoneally. The derivative was injected subcutaneously immediately after the inoculation. The $CD_{50}$ value in male ddy mice (Shizuoka) was 0.55 mg/kg.

J) Toxicity

No acute toxicity was observed in male ddy mice to which the N-desacetyl derivative was administered intraperitoneally at a dose of 500 mg/kg.

Example 2

Two-hundred mg of triethyl ester of the antibiotic PS—5 prepared according to Referential Example 2 was dissolved in 10 ml of dichlormethane. With ice-cooling, 5 ml of pyridine was added to the solution, to which 1 g of phosphorus pentachloride was added with stirring. The mixture was kept at the same temperature with stirring for 30 minutes and cooled with dry ice-acetone. After 5 ml of methanol was added, the mixture was stirred for 1 hour. To the mixture was poured a mixture of 200 ml of 0.1 M phosphate buffer solution, pH 6.8, and 200 ml of methanol to form a homogeneous solution. A further procedure similar to the one described in Example 1 provided 4.2 mg of N-desacetyl derivative of the antibiotic PS—5.

Example 3

Fifty milliliters of a medium in 250 ml-Erlenmeyer flask consisting of 2% glucose, 0.8% Pharmamedia and 0.5% corn steep liquor was inoculated with *Protaminobacter ruber* NRRL B—8143, deposited November 11, 1976, and incubated at 28°C for 24 hours as a seed culture. To five 500 ml-Erlenmeyer flasks containing 100 ml each of the same medium was added 3 ml each of the seed culture. After shaking culture was accomplished at 28°C for about 100 hours, the culture broths were combined.

Centrifugation at 8000 r.p.m. for 10 minutes at 0°C provided a precipitate containing cells, which was suspended in 0.01 M phosphate buffer solution, pH 7.4. The suspension was allowed to stand at 5°C for 30 minutes and the resulting precipitate consisting of solids in the medium was removed. The cells were recovered by centrifuging the upper cell suspension layer at 8000 r.p.m. for 10 minutes at 0°C. The recovered cells were suspended in 5 ml of 0.01 M phosphate buffer solution, pH 7.4, and subjected to sonication with an ultrasonic cell disruption apparatus (Tomy Seiko Co. Ultrasonifier UP—200p) at 60 W for 2 minutes to disrupt them. The product was centrifuged at 10,000 r.p.m. for 15 minutes at 0°C to remove the cell debris to obtain a cell-free extract. A mixture consisting of 0.3 ml of the cell-free extract, 0.2 ml of 0.1 M phosphate buffer solution, pH 7.4 0.1 ml of 10 mM $CoCl_2 \cdot 6H_2C$ solution and 0.4 ml of substrate solution containing 28 μg of the antibiotic PS—5 prepared according to Referential Example 1 was incubated at 28°C for 3 hours. A 20 μl portion of the reaction mixture was spotted on a sheet of Whatman No. 1 filter paper. Paper electrophoresis was carried out in a buffer solution, pH 8.6 (consisting of 3000 ml of water, 3.3 g of barbital and 25.5 g of sodium barbital) with a charge of 42 V/cm at 4°C for 30 minutes. The filter paper was dried and subjected to bioautography on a *Staphylococcus* assay plate. A growth inhibitory zone caused by the reaction product, N-desacetyl derivative of the antibiotic PS—5, was detected 4 mm from the origin toward a cathode. The unreacted PS—5 gave an inhibitory zone 30 mm from the origin toward an anode. The yield of the reaction was about 7%. A 20 μl portion of the reaction mixture was spotted on a sheet of Toyo filter paper No. 50 and subjected to descending paper chromatography with a solvent system consisting of 120 ml of

acetonitrile, 30 ml of M/10 tris-(hydroxymethyl)aminomethane-hydrochloric acid buffer solution, pH 7.5 and 1 ml of M/10 aqueous sodium ethylene-diamine tetraacetate, pH 7.5, and the bioautography was performed with the *Staphylococcus* assay plate. The reaction product, N-desacetyl derivative of the antibiotic PS—5, showed the growth inhibitory zone at RF 0.22 and the unreacted PS—5 showed it at Rf 0.34.

The control in which distilled water was used in place of the cell-free extract gave a growth inhibitory zone only at the Rf value of the antibiotic PS—5.

As described above, a pharmaceutical preparation containing the compound of formula (I) may be administered in various unit dosage forms such as in solid or liquid orally ingestible dosage form. The preparation per unit dosage, whether solid or liquid, may contain the active material in an amount of 0.1—99%, preferably 10—60%. The amount of the active ingredient in the preparation may change depending on the dosage form and the total weight of the preparation, and usually is in the range of 10 mg to 1,000 mg, preferably 100 mg to 1,000 mg.

In parenteral administration, the formulation of the unit dose is usually a solution of about 100% pure compound of formula (I) in sterilized water or a soluble powder for solution.

Representative formulations containing the compound of formula (I) according to this invention are as follows:

Example A: CAPSULES

| Component | Per capsule |
|---|---|
| Compound of formula (I) | 100 mg |
| Lactose (J.P.)* | a sufficient number |
| Magnesium stearate | 1 mg |

*J.P. = Japanese Pharmacopoeia.

The active compound and the diluents are mixed well in a mortar with a pestle to produce a uniform blend. Two hundred milligrams of the blend is filled in a No. 3 hard gelatin capsule.

Example B: TABLETS

| Component | Per tablet |
|---|---|
| Compound of formula (I) | 200 mg |
| Lactose (J. P.) | 120 mg |
| Corn starch | 175 mg |
| Magnesium stearate | 5 mg |
| | 500 mg |

In the above composition, the active component is blended with lactose and a half amount of corn starch. The mixture is granulated with 10% paste of the above amount of corn starch and screened. Another half amount of corn starch and magnesium stearate are added and the mixture is compressed into tablets, approximately 1 cm in diameter, weighing 500 mg. The tablets are sugar-coated and then enteric-coated.

Example C: LYO FORM FOR INJECTION

| Component | Per vial |
|---|---|
| Compound of formula (I) | 25 mg |
| Sterilized distilled water for injection (J.P.) | 2 ml |

The active component is dissolved in sterilized distilled water for injection, filtered and sterilized. The solution is subdivided into sterilized vials, and water is aseptically removed by lyophilization. The vials containing the sterilized dry solid are aseptically sealed.

For administration, 2 ml of sterilized physiological saline (J.P.) is added to the contents of a vial.

Example D: TABLETS

| Component | Per tablet |
|---|---|
| Compound of formula (I) | 20 mg |
| Cephaloridine | 180 mg |
| Lactose (J.P.) | 120 mg |
| Corn starch | 175 mg |
| Magnesium stearate | 5 mg |
| | 500 mg |

The compound of formula (I) is mixed with Cephaloridine, and processed and compressed into tablets as described in Example B. The tablets are sugar-coated and then enteric-coated.

Example E: TABLETS

| Component | Per tablet |
|---|---|
| Compound of formula (I) | 10 mg |
| Aminobenzylpenicillin | 190 mg |
| Lactose (J.P.) | 120 mg |
| Corn starch | 175 mg |
| Magnesium stearate | 5 mg |
| | 500 mg |

By the same method as described in Example B, the compound of formula (I) and aminobenzylpenicillin are mixed and processed.

**Claims**

1. A compound of the formula

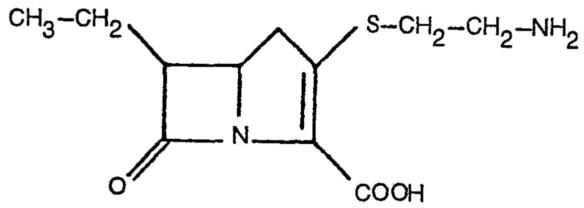

(I)

having 5,6-trans-stereo configuration.

2. A process for producing a compound of the formula

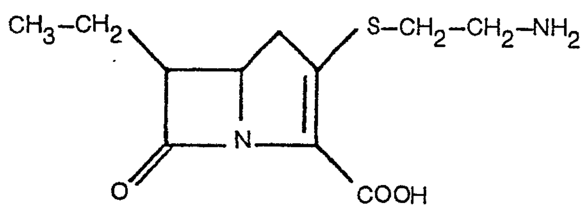

(I)

or a carboxyl-protected derivative thereof, which comprises chemically or biologically cleaving off the N-acetyl group from a compound of the formula

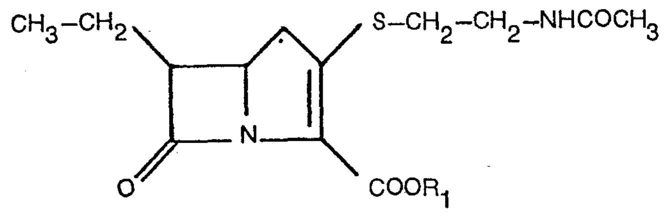

(II)

wherein $R_1$ represents a hydrogen atom, a carboxyl-protecting group, or a salt thereof when $R_1$ is a hydrogen atom, and if desired, splitting-off the carboxyl-protecting group, which may be present, from the reaction product.

3. The process of claim 2, in which the compound of the formula (I) or the carboxyl-protected derivative thereof is prepared by reacting a compound of the formula

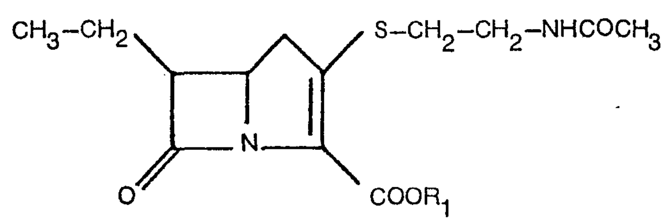

(II)

wherein $R_1$ represents a carboxyl-protecting group, with an iminohalogenating agent, and reacting the resulting imino-halide compound with an alcohol of the formula

$$R_2OH \qquad (III)$$

wherein $R_2$ represents a hydrocarbon residue, followed by treatment with water, and if desired, splitting off the carboxyl-protecting group, which may be present, from the reaction product.

4. The process of claim 3 in which the iminohalogenating agent is selected from phosphorus pentachloride, phosphorus pentabromide, phosphorus trichloride, phosphorus tribromide, phosphorus oxychloride, phosphorus oxybromide, thionyl chloride, thionyl bromide and phosgene.

5. The process of claim 3 in which the reaction between the compound of formula (II) and the iminohalogenating agent is carried out in an inert organic solvent at room temperature or at a lower temperature.

6. The process of claim 3 in which 1 to 20 equivalents of the iminohalogenating agent is reacted per mole of the compound of formula (II).

7. The process of claim 3 in which the reaction of the compound of formula (II) with the iminohalogenating agent is carried out in the presence of a base.

8. The process of claim 3 in which the alcohol is a substantially anhydrous lower alkanol.

9. The process of claim 3 in which the reaction between the iminohalide compound and the alcohol is carried out at a temperature below room temperature.

10. The process of claim 3 in which the treatment with water is carried out at a temperature below room temperature using an aqueous buffer solution having a pH of 5 to 9.

11. The process of claim 2 in which the compound of the formula (I) is prepared by treating a compound of the formula

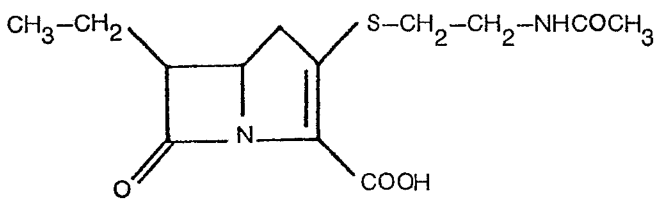

(IV)

or its salt with microbial cells or their treated product having the ability to hydrolyze the N-acetyl group.

12. The method of claim 11 in which the microbial cells are those of *Protaminobacter ruber* NRRL B—8143 (FERM P—3794) deposited November 11, 1976.

13. A pharmaceutical composition having an antimicrobial activity comprising, as an active ingredient, a compound of the formula

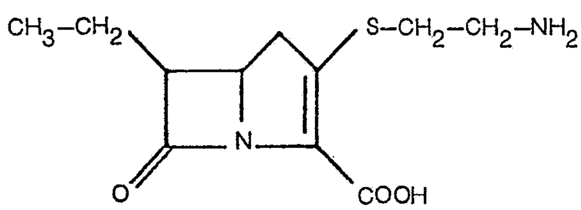

(I)

and a suitable carrier material.

14. A compound of the formula

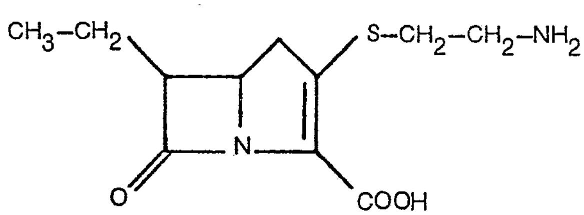

(I)

for use in combatting or preventing a bacterial infection in an animal.

**Patentansprüche**

1. Verbindung der Formel

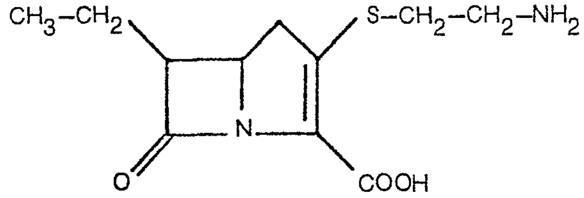

(I)

mit 5,6-trans-Stereo-Konfiguration.

2. Verfahren zur Herstellung einer Verbindung der Formel

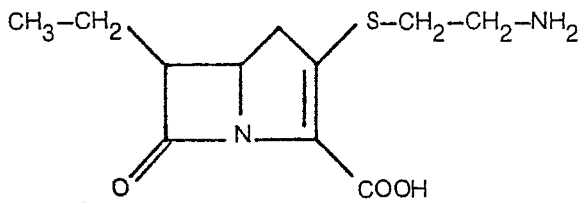

(I)

oder eines ihrer carboxylgeschützten Derivate, dadurch gekennzeichnet, daß man chemisch oder biologisch die N-Acetylgruppe von einer Verbindung der Formel

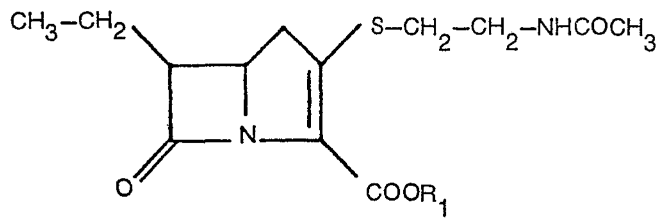

(II)

worin $R_1$ ein Wasserstoffatom, eine Carboxylschutzgruppe bedeutet oder von einem Salz davon, wenn $R_1$ ein Wasserstoffatom bedeutet, abspaltet und gegebenenfalls die Carboxylschutzgruppe, die vorhanden sein kann, aus dem Reaktionsprodukt abspaltet.

3. Verfahren nach Anspruch 2, dadurch gekennzeichnet, daß die Verbindung der Formel (I) oder ihr carboxylgeschütztes Derivat durch Umsetzung einer Verbindung der Formel

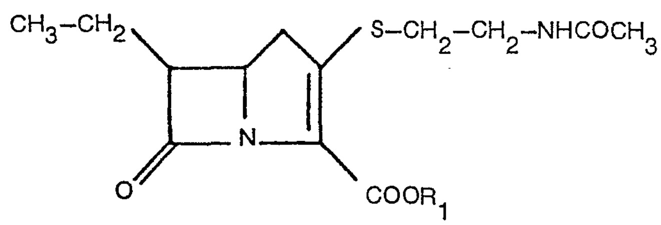

(II)

worin $R_1$ eine Carboxylschutzgruppe bedeutet, mit einem Iminohalogenierungsmittel und durch Umsetzung der entstehenden Iminohalogenidverbindung mit einem Alkohol der Formel

$R_2OH$ (III)

worin $R_2$ einen Kohlenwasserstoffrest bedeutet, und anschließende Behandlung mit Wasser und gegebenenfalls Abspaltung der Carboxylschutzgruppe, die vorhanden sein kann, aus dem Reaktionsprodukt hergestellt wird.

4. Verfahren nach Anspruch 3, dadurch gekennzeichnet, daß das Iminohalogenierungsmittel ausgewählt wird unter Phosphorpentachlorid, Phosphorpentabromid, Phosphortrichlorid, Phosphortribromid, Phosphoroxychlorid, Phosphoroxybromid, Thionylchlorid, Thionylbromid und Phosgen.

5. Verfahren nach Anspruch 3, dadurch gekennzeichnet, daß die Reaktion zwischen der Verbindung der Formel (II) und dem Iminohalogenierungsmittel in einem inerten organischen Lösungsmittel bei Raumtemperatur oder bei einer niedrigeren Temperatur durchgeführt wird.

6. Verfahren nach Anspruch 3, dadurch gekennzeichnet, daß 1 bis 20 Äquivalente Iminohalogenierungsmittel pro Mol der Verbindung der Formel (II) umgesetzt werden.

7. Verfahren nach Anspruch 3, dadurch gekennzeichnet, daß die Reaktion der Verbindung der Formel (II) mit dem Iminohalogenierungsmittel in Anwesenheit einer Base durchgeführt wird.

8. Verfahren nach Anspruch 3, dadurch gekennzeichnet, daß man als Alkohol ein im wesentlichen wasserfreies niedriges Alkanol verwendet.

9. Verfahren nach Anspruch 3, dadurch gekennzeichnet, daß die Reaktion zwischen der Iminohalogenidverbindung und dem Alkohol bei einer Temperatur unter Raumtemperatur durchgeführt wird.

10. Verfahren nach Anspruch 3, dadurch gekennzeichnet, daß die Behandlung mit Wasser bei einer Temperatur unter Raumtemperatur unter Verwendung einer wäßrigen Pufferlösung mit einem pH-Wert von 5 bis 9 durchgeführt wird.

11. Verfahren nach Anspruch 2, dadurch gekennzeichnet, daß die Verbindung der Formel (I) durch Behandlung einer Verbindung der Formel

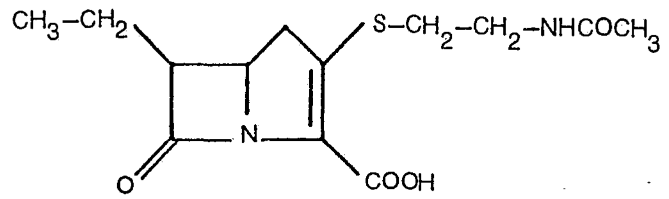

(IV)

oder ihres Salzes mit Mikrobenzellen oder ihrem behandelten Produkt, das die Fähigkeit hat, die N-Acetylgruppe zu hydrolysieren, hergestellt wird.

12. Verfahren nach Anspruch 11, dadurch gekennzeichnet, daß als Mikrobenzellen solche von *Protaminobacter ruber* NRRL B—8143 (FERM P—3794), hinterlegt am 11. November 1976, verwendet werden.

13. Pharmazeutische Zubereitung mit antimikrobieller Aktivität, dadurch gekennzeichnet, daß sie als aktiven Bestandteil eine Verbindung der Formel

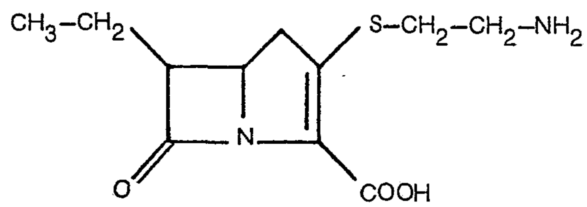

(I)

und ein geeignetes Trägermaterial enthält.

14. Verbindung der Formel

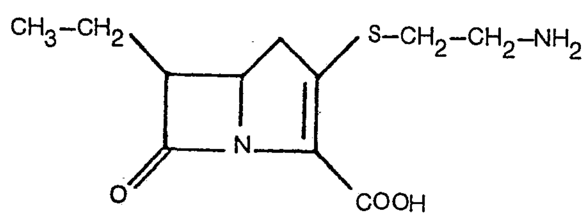

(I)

für die Verwendung bei der Bekämpfung oder Prophylaxe einer Bakterieninfektion in einem Tier.

**Revendications**

1. Composé de formule

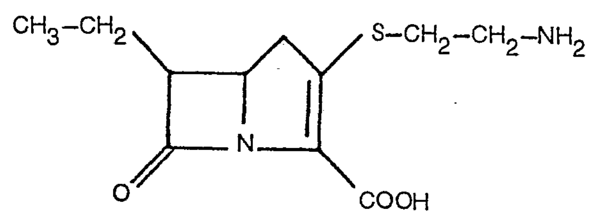

(I)

ayant la stéréo-configuration 5,6-trans.

2. Procédé de production du composé de formule

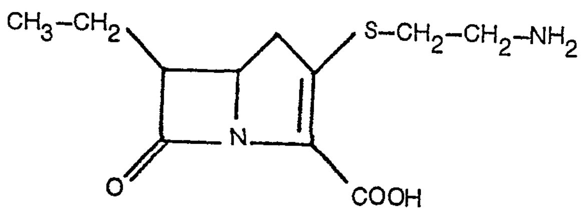

(I)

ou d'un dérivé de celui-ci dont la fonction carboxylique est protégée, procédé selon lequel on élimine par voie chimique ou biologique le groupe N-acétylique du composé de formule

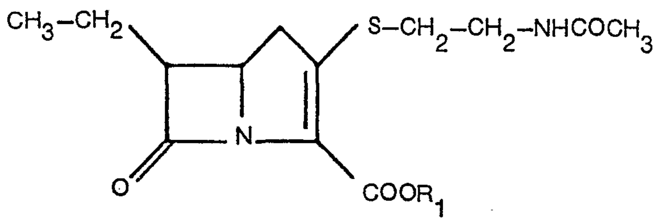

(II)

$R_1$ représentant un atome d'hydrogène, un groupe protégeant la fonction carboxylique ou un sel de celle-ci si $R_1$ est un atome d'hydrogène, et le cas échéant on élimine le groupe protecteur $R_1$.

3. Procédé selon la revendication 2 dans lequel le composé de formule I ou son dérivé à fonction carboxylique protégée est préparé par réaction du composé de formule

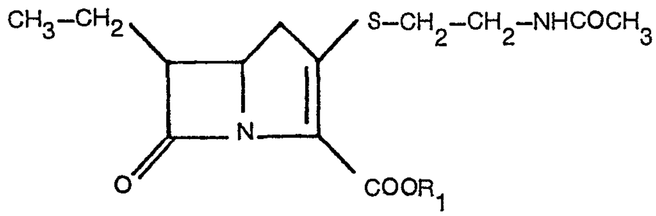

(II)

$R_1$ représentant un groupe protégeant la fonction carboxylique, avec un agent d'iminohalogénation, puis réaction de l'iminohalogénure formé avec un alcool de formule

$R_2OH$ (III)

$R_2$ représentant un radical hydrocarboné, suivie d'un traitement à l'eau, et le cas échéant on élimine le groupe protecteur $R_1$.

4. Procédé selon la revendication 3 dans lequel l'agent iminohalogénant est choisi parmi le pentachlorure, le pentabromure, le trichlorure, le tribromure, l'oxychlorure et l'oxybromure de phosphore, le chlorure de thionyle, le bromure de thionyle et le phosgène.

5. Procédé selon la revendication 3 ou 4 dans lequel la réaction entre le composé de formule II et l'agent iminohalogénant est effectuée dans un solvant organique inerte, à la température ordinaire ou à une température inférieure.

6. Procédé selon l'une quelconque des revendications 3 à 5 dans lequel on fait réagir 1 à 20 équivalents de l'agent iminohalogénant par mole du composé de formule II.

7. Procédé selon l'une quelconque des revendications 3 à 6 dans lequel la réaction du composé de formule II avec l'agent iminohalogénant est effectuée en présence d'une base.

8. Procédé selon l'une quelconque des revendications 3 à 7 dans lequel l'alcool est un alcanol inférieur sensiblement anhydre.

9. Procédé selon l'une quelconque des revendications 3 à 8 dans lequel la réaction entre l'iminohélogénure et l'alcool est effectuée à une température inférieure à la température ordinaire.

10. Procédé selon l'une quelconque des revendications 3 à 9 dans lequel le traitement à l'eau est effectué à une température inférieure à la température ordinaire, avec une solution aqueuse tamponnée à un pH 5 à 9.

11. Procédé selon la revendication 2 dans lequel le composé de formule I est préparé par traitement du composé de formule

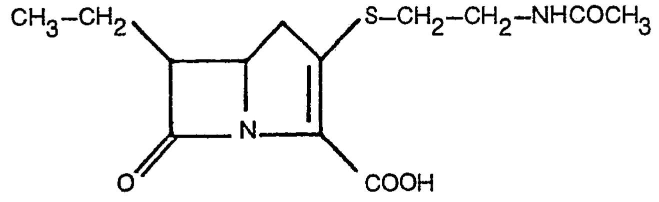

(IV)

ou d'un sel de celui-ci avec des cellules microbiennes ou un produit résultant du traitement de ces cellules, pouvant hydrolyser le groupe N-acétylique.

12. Procédé selon la revendication 11 dans lequel les cellules microbiennes sont des cellules de la souche Protaminobacter ruber NRRL B—8143 (FERM P—3794) déposée le 11 Novembre 1976.

13. Composition pharmaceutique ayant notamment une action anti-microbienne, comprenant comme ingrédient actif le composé de formule

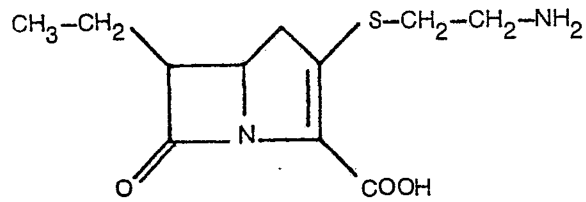

(I)

avec un véhicule approprié.

14. L'utilisation du composé de formule

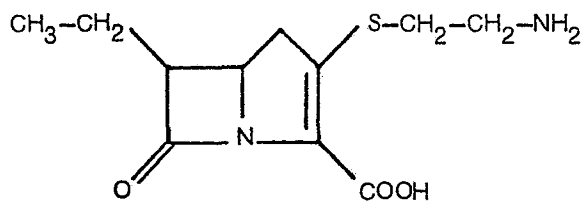

(I)

pour combattre ou prévenir des infections bactériennes chez l'homme ou l'animal.

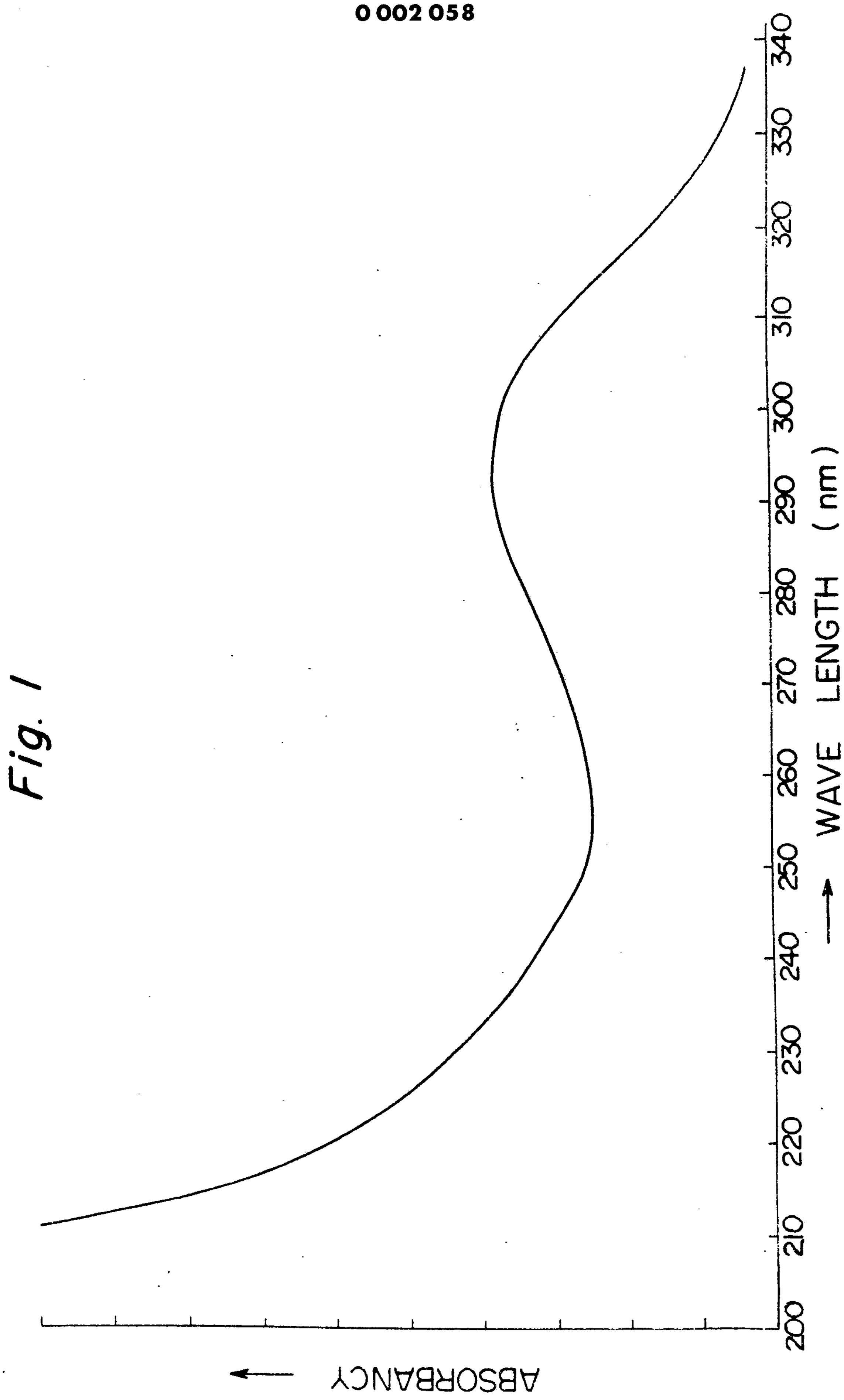
200
210
220
230
240
250
260
270
280
290
300
310
320
330
340
→ WAVE LENGTH (nm)
ABSORBANCY →

Fig. 1

Fig. 2

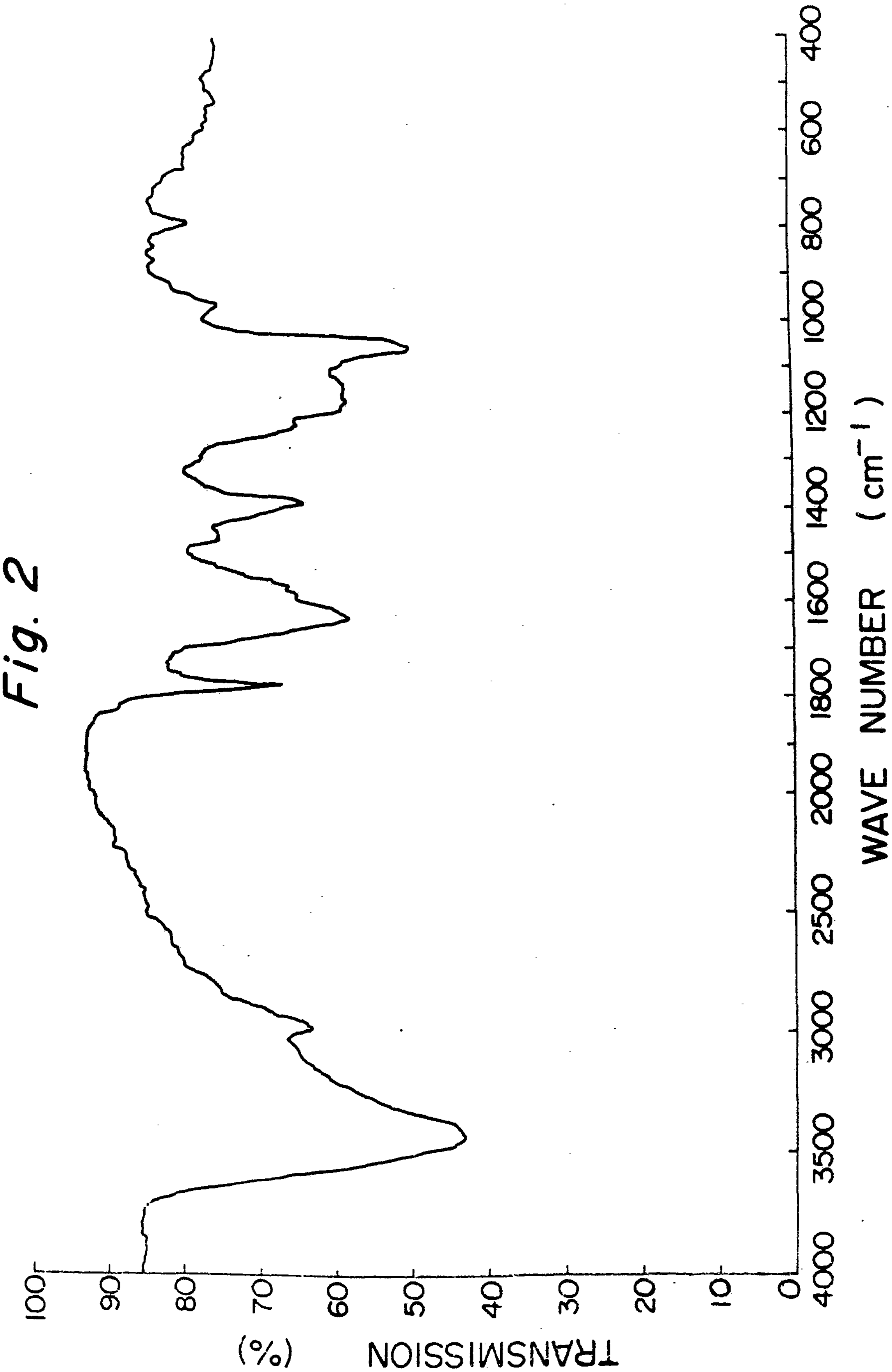
TRANSMISSION (%)
100
90
80
70
60
50
40
30
20
10
0
4000
3500
3000
2500
2000
1800
1600
1400
1200
1000
800
600
400
WAVE NUMBER (cm⁻¹)